# EUROPEAN PATENT APPLICATION

(11) **EP 3 421 456 A1**
(43) Date of publication of application: **02.01.2019**
(21) Application number: 18179051.0
(22) Date of filing: 21.06.2018
(51) Int. Cl.: C07D 213/81, C07D 413/04, A61P 25/16, A61K 31/4412

(54) **NEW ROUTE OF SYNTHESIS FOR OPICAPONE**

(30) Priority: 27.06.2017 GB 201710241
(71) Applicant: AZAD Pharmaceutical Ingredients AG, 8200 Schaffhausen (CH)
(72) Inventor: FRECH NABOLD, Christian, 8614 Bertschikon (CH); AEBERSOLD, Christine, 8610 Uster (CH); GRIECO, Gabriele, 20094 Corsico (IT); GERBER AESCHBACHER, Roman, 8500 Frauenfeld (CH)
(74) Representative: Michalski Hüttermann & Partner Patentanwälte mbB

(57) **Abstract**

The present invention relates to a new route of synthesis for obtaining Opicapone ((4Z)-4-[3-(2,5-dichloro-4,6-dimethyl-1-oxidopyridin-1-ium-3-yl)-2H-1,2,4-oxadia-zol-5-ylidene]-2-hydroxy-6-nitrocyclohexa-2,5-dien-1-one), new intermediates in the reaction path and the new substance 2,5-dichloro-N-hydroxy-4,6-dimethylpyridine-3-carboximidoyl chloride.

## Description

### FIELD OF THE INVENTION

The present invention relates to a new route of synthesis for obtaining Opicapone ((4Z)-4-[3-(2,5-dichloro-4,6-dimethyl-1-oxidopyridin-1-ium-3-yl)-2H-1,2,4-oxadia-zol-5-ylidene]-2-hydroxy-6-nitrocyclohexa-2,5-dien-1-one), new intermediates in the reaction path and the new substance 2,5-dichloro-N-hydroxy-4,6-dimethylpyridine-3-carboximidoyl chloride.

### BACKGROUND

The current gold standard in the treatment of Parkinson's disease is the administration of levodopa (L-DOPA). Unfortunately, the half-life of L-DOPA is very short due to the *in vivo* O-methylation of L-DOPA to 3-O-methyl-L-DOPA (3-OMD) caused by catechol-O-methyltransferase (COMT)-enzymes. The *in vivo* half-life of 3-OMD is much longer compared to L-DOPA and, in addition, both molecules compete for the same transport-route across the blood-brain-barrier, resulting in a very low concentration of systemically administered L-DOPA in the brain and accumulation of 3-OMD. In consequence, after a certain time period the administration of L-DOPA alone becomes ineffective and the overall treatment becomes unable to improve the clinical symptoms resulting in motor fluctuations.

One way to prevent such "wearing-off" phenomenon is a combination treatment of L-DOPA and a COMT-inhibitor. The COMT inhibitor protects L-DOPA *in vivo* from O-methylation and this result in much higher L-DOPA concentrations in the brain. In addition, the "wearing-off" phenomenon is reduced, because less 3-OMD is accumulated in the body.

Several potent COMT inhibitors like Talcapone or Entacapone are suggested for the combination treatment, wherein the inhibitors differ in their penetration ability to the central nervous systems (CNS) and hepatotoxicity.

Furthermore, nitrocatechols for COMT inhibition with low cytotoxicity are for instance disclosed in EP 1 907 382 B1. Opicapone is one representative of this compound class and the patent document discloses that the chemical functionality of the non-catecholic substituent connected to the heterocyclic ring determines the toxicity-profile. Besides the advantageous toxicological properties of this compound class also several routes of synthesis are disclosed in EP 1 907 382 B1.

Nevertheless, besides the published ways of production there is still the need for new synthesis routes which are able to provide Opicapone in pharmaceutically acceptable quality in high yields and in environmentally friendly processes.

It is therefore an object of the present invention to provide such new route of synthesis for the production of pharmaceutically acceptable Opicapone, pharmaceutical compositions comprising the same and the use of the pharmaceutical compositions for the treatment of central nervous disorders. In addition, it is a further object of the present invention to disclose new intermediates in the route of synthesis and a new chemical compound.

### BRIEF DESCRIPTION OF THE INVENTION

Above mentioned task is solved by a process for the preparation of Opicapone ((4Z)-4-[3-(2,5-dichloro-4,6-dimethyl-1-oxidopyridin-1-ium-3-yl)-2H-1,2,4-oxadiazol-5-ylidene]-2-hydroxy-6-nitrocyclohexa-2,5-dien-1-one) according to the following formula 11 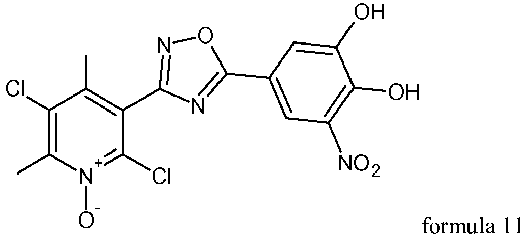 or a pharmaceutically acceptable salt thereof, wherein the process at least comprises the reaction of a compound according to the following formula 6 (2,5-dichloro-N-hydroxy-4,6-dimethylnicotinimidoyl chloride) 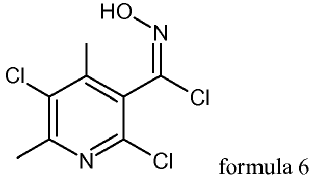 and a compound according to the following formula 8 (4-hydroxy-3-methoxy-5-nitrobenzonitrile) 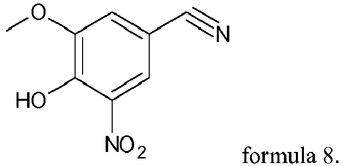 in the presence of a solvent and a base. Surprisingly, it has been found that above given route of synthesis is able to build the central Opicapone ring structure in high yields, high purity and only a small amount of side-products which can easily be separated from the desired central Opicapone ring structure by standard purification techniques. Within this reaction the pyridine-ring structure is part of the educt according to formula 6, the benzene-ring is part of the educt according to formula 8 and the central oxadiazol-ring is formed in the course of reaction between the functional groups of the two educts. The advantages in processing can be attributed, at least in part, to the overall gentle processing conditions compared to other state-of the art processes. The obtainable central Opicapone ring structure is easily transferred to Opicapone by only two process steps, rendering the overall route of synthesis efficient and reproducible. It is especially surprising that this ring formation is possible, because other theoretically feasible ring forming reactions like the reaction of a compound according to formula 6 with a base to yield the nitrile-oxide followed by reaction with compound 8 failed and no product could be synthesized. Furthermore, it is advantageous in the presented process that the two compounds which form the central Opicapone ring structure can also be obtained in high yields at very gentle processing conditions, wherein it is very remarkable that the reactions for the synthesis of above displayed educts can be performed at low temperatures. Especially the latter reduces the quantity of unwanted side-products and reduces the necessity and amount of extensive purification steps, rendering the overall process very cost efficient.

Opicapone can be obtained as such as but is also possible to provide the substance in form of a pharmaceutically acceptable salt. For instance, it is possible to extract a product of the hydroxyl group and achieve an alkoxide salt. Feasible counter-ions can for instance be alkali or alkaline earth ions.

The reaction between the compound according to formula 6 and the compound according to formula 8 is performed in a suitable solvent. The solvent can be any pharmaceutically acceptable solvents, wherein aprotic solvents are preferred. Suitable solvents can for instance be benzene, xylene, dioxane, DMF, THF, DCM, chloroform or mixtures thereof.

Surprisingly it has been found that the presence of a base drastically reduces the necessary reaction times. Suitable bases can either be organic or inorganic bases. Possible bases are for instance DIPEA or other tertiary amines, but also secondary amines and K₂CO₃ or other carbonates, NaOH, tBuOK, NaHCO₃ or Na₃PO₄.

In a preferred embodiment of the process the reaction of the compound according to formula 6 and formula 8 can be performed at a temperature of ≥ 80°C and ≤ 140°C. Within this temperature range it is possible to achieve high reaction rates and surprisingly it was found that under these temperature conditions the amount of unwanted side-products is reduced compared to reaction performed below the given temperature range. Therefore, it is possible to obtain the desired product in short reaction times in high purity. Furthermore, it is preferred to perform the reaction at temperatures of ≥ 90°C and ≤ 130°C, preferably at temperatures of ≥ 100°C and ≤ 120°C.

In a further characteristic of the process the reaction of the compound according to formula 6 and formula 8 can be performed in one or more aromatic solvents comprising a boiling point of ≥ 80°C. It has been found that especially in aromatic solvents comprising a high boiling point the generation of unwanted byproducts is reduced and high yields are obtainable within short reaction times. A preferred solvent for the reaction is toluene.

Within another aspect of the process the base can be a tertiary amine. Especially tertiary amines are very suitable to catalyze the formation of the central Opicapone ring structure. The achievable reaction rates are higher compared to other bases and it was surprisingly found that especially in contrast to prim. or secondary amines the amount of side-products is significantly reduced. Without being bound by the theory this might be attributed to the better solubility of the organic tert. amines in aromatic solvents. Furthermore, it is advantageous that the protonated tertiary amines precipitate and the precipitate can easily be removed from the reaction solution further driving the reaction to the product side.

In a further embodiment of the process the tertiary amine can be NEt₃. Especially triethylamine is able to provide high reaction rates and a very low amount of unwanted side-products. Furthermore, it is advantageous to add the trimethylamine step wise to the solvent. This procedure additionally reduces the formation of unwanted by-products.

In a preferred embodiment of the process the reaction product of the reaction between a compound according to formula 6 and a compound according to formula 8 can be a compound according to formula 9 (4-(3-(2,5-dichloro-4,6-dimethylpyridin-3-yl)-1,2,4-oxadiazol-5-yl)-2-methoxy-6-nitrophenol) 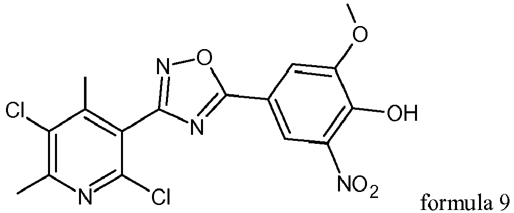 and the compound according to formula 9 can be de-methylated to yield a compound according to formula 10 (5-(3-(2,5-dichloro-4,6-dimethylpyridin-3-yl)-1,2,4-oxadiazol-5-yl)-3-nitrobenzene-1,2-diol) 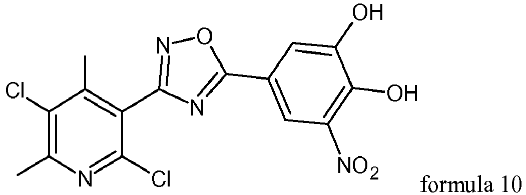 followed by N-oxidation to yield Opicapone according to formula 11. Especially the above disclosed route of synthesis, wherein the compound according to formula 9 is firstly de-methylated and oxidized afterwards, provides very high yields and a very low amount of unwanted by-products compared to a route of reaction, wherein both process steps are reversed. For the oxidation reaction any pharmaceutically acceptable oxidation reagent can be used. Especially preferred oxidation agents can be selected from the group consisting of hydrogen peroxide, Urea hydrogen peroxide, NaIO₄, KIO₄, NR₄IO₄, Ba(IO₄)₂, Na₃H₂IO₆, orthoiodates (IO₆⁵⁻), metaperiodates, ozone, wherein hydrogen peroxide is preferred.

In another aspect of the process the oxidation step from the compound according to formula 10 to the compound according to formula 11 can be performed in the presence of hydrogen peroxide and trifluoroacetic anhydride at room temperature. The oxidation of the compound according to formula 10 to Opicapone can be readily achieved in trifluoroacetic anhydride as a solvent. The reaction is quantitative and the reaction times are short.

Within a further characteristic of the process the oxidation step from the compound according to formula 10 to the compound according to formula 11 can be performed in dichloromethane, chloroform or mixtures thereof. It has been found useful to perform the oxidation step in the presence of chlorinated solvents and especially in the presence of above mentioned solvents or solvent mixtures. Also within these solvent high yields for this step are obtainable and the purification of the product can easily be performed. In the explicitly mentioned solvents or solvent mixture the oxidation step is possible directly at ambient temperature.

Within a further, preferred embodiment of the process the compound according to formula 6 can be prepared via the steps of
a) reacting 4,6-dimethyl-2-hydroxynicotinonitrile and a chlorinating agent to yield 2,5-dichloro-4,6-dimethylnicotinonitrile 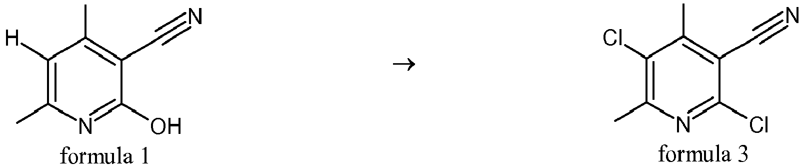
b) reacting the 2,5-dichloro-4,6-dimethylnicotinonitrile obtained in step a) in the presence of a reducing agent to yield 2,5-dichloro-4,6-dimethylnicotin-aldehyde 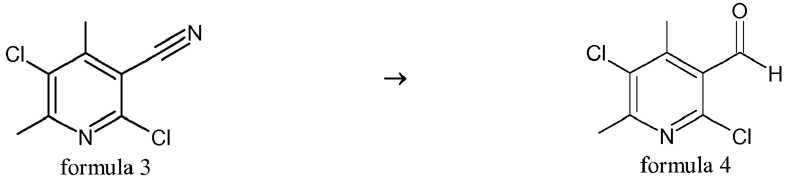
c) reacting the 2,5-dichloro-4,6-dimethylnicotinaldehyde obtained in step b) with hydroxylamine to yield 2,5-dichloro-4,6-dimethylnicotinaldehyde-oxime 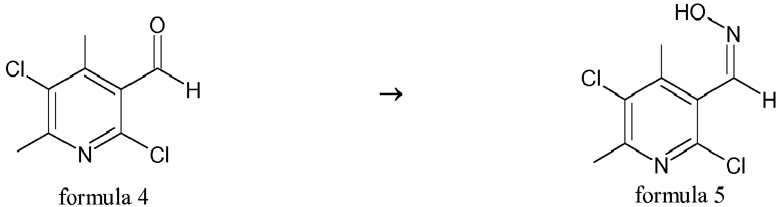
d) reacting the 2,5-dichloro-4,6-dimethylnicotinaldehyde-oxime obtained in step c) and a chlorinating agent to yield compound 5 2,5-dichloro-N-hydroxy-4,6-dimethylnicotinimidoyl-chloride

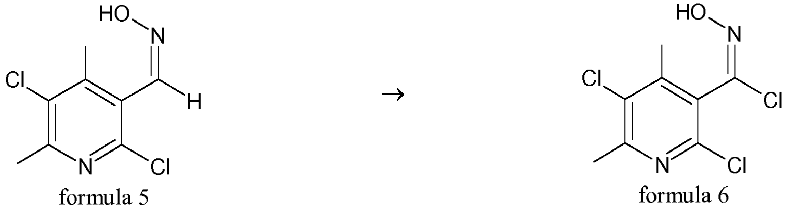

This route of synthesis is able to provide the compound according to formula 6 in high yields and each synthesis step can be performed at very moderate reaction conditions. Although all reaction steps can for instance be performed at ambient temperature, high yields are obtainable at very short reaction times and the moderate synthesis conditions do also favor selective product formation, resulting in a very low amount of side-products.

In another aspect of the process the chlorinating step a) can be performed stepwise, wherein in a first reaction step
a1) 4,6-dimethyl-2-hydroxynicotinonitrile is mono-chlorinated in 5 position in the presence of sulfuryl chloride to yield 5-chloro-2-hydroxy-4,6-dimethylnicotinonitrile 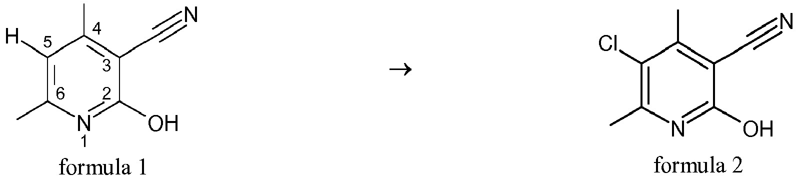 and in a second step
a2) 5-chloro-2-hydroxy-4,6-dimethylnicotinonitrile is chlorinated in the presence of phosphorus oxychloride to 2,5-chloro-4,6-dimethylnicotinonitrile

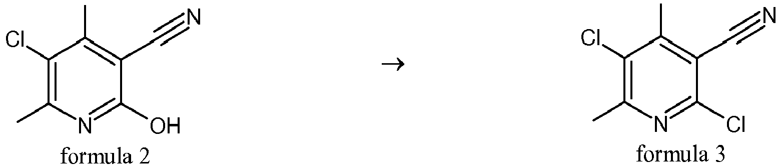

It has been found favorable to perform the chlorination of the compound according to formula 1 in a two-step process, wherein in a first reaction step the hydrogen atom in 5-position and, in a second step, the hydroxyl in 2-position is substituted. This two-step reaction is much better controllable compared to a reaction, wherein the hydrogen-atom and the hydroxyl-group are substituted at once. Furthermore, the two-step reaction can be performed at very gentle process condition. Especially, the temperature can be keep low, which results in the formation of a very clean product.

Within a preferred embodiment of the process the compound according to formula 8 can be prepared via reaction of 5-nitrovaniline and hydroxylamine hydrochloride to yield the compound according to formula 8

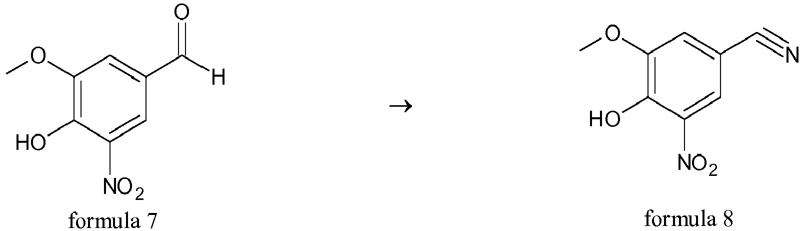

Also for the proposed route of preparation for the nitrile high yields are obtainable at very moderate reaction temperatures. As described above for the preparation of the compound according to formula 6 this route enables a very energy efficient synthesis and results in a product comprising only very low concentrations of side-products.

In another aspect of the process all reaction steps in the preparation of the compound according to formula 6 and all reaction steps in the preparation of the compound according to formula 8 can be performed below 50°C. One of the major advantages of the proposed route of synthesis to yield the intermediates according to formulae 6 and 8 is the possibility to achieve high yields at short reaction times even at very moderate reaction conditions. This renders the reaction very controllable and energy efficient.

It is further within the scope of invention to disclose the compound (2,5-dichloro-N-hydroxy-4,6-dimethylpyridine-3-carboximidoyl chloride) according to the following formula 6 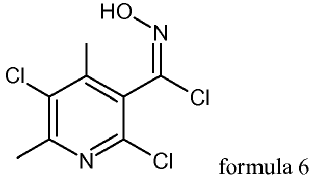 or a salt thereof. The salt form of the compound may for instance be generated by de-protonation of the OH-group with a suitable base, wherein the cation of the base generates the salt form of the compound according to formula 6. Suitable bases may be sodium or potassium hydroxide. In consequence, the sodium or potassium salts of the compound according to formula 6 are achieved. Possible cations can be metal cations, wherein it is known to the skilled artisan that the stoichiometry between anion and cation is not necessarily 1 to 1, depending on the charges. In addition, it is also possible to use the compound according to formula 6 in the form, wherein the cation is a metal-ligand complex.

In addition, it is within the scope of invention to disclose an intermediate in the production of Opicapone at least comprising a compound according to the following formula 6 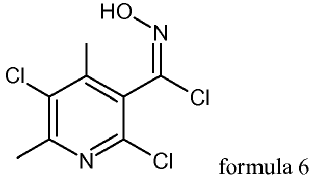 or a pharmaceutically acceptable salt thereof. For the generation of pharmaceutically acceptable salts for instance pharmaceutically acceptable bases can be used as mentioned in the pharmacopoeia. Suitable are for instance the alkaline or alkaline earth bases, but also for instance amines.

Furthermore, another intermediate in the production of Opicapone can at least comprise a compound according to the following formula 8 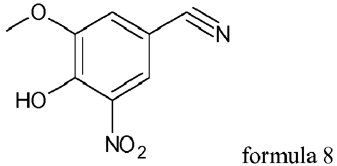 or a pharmaceutically acceptable salt thereof. For the generation of the pharmaceutically acceptable salts the same bases as mention for the compounds according to formula 6 can be used.

In addition, a pharmaceutical composition comprising Opicapone synthesized according to the inventive process in combination with a pharmaceutically acceptable carrier is within the scope of invention. These pharmaceutical compositions comprise at least the inventively synthesized Opicapone as an API (Active Pharmaceutical Ingredient) and optionally further pharmaceutical acceptable excipients. The inventively achievable Opicapone is especially suitable for use in a pharmaceutical composition because the Opicapone can be processed in a more reproducible way compared to routes of synthesis and superior quality. Hence, pharmaceutical compositions are accessible comprising more homogeneous characteristics.

In addition, the use of a pharmaceutical composition comprising Opicapone synthesized according to the inventive process for the treatment of central or peripheral nervous system disorders is within the scope of invention. Especially, a combination treatment of L-DOPA and the inventively synthesized Opicapone is suitable to reduce the symptoms or above mentioned disorders.

### BRIEF DESCRIPTION OF THE DRAWINGS

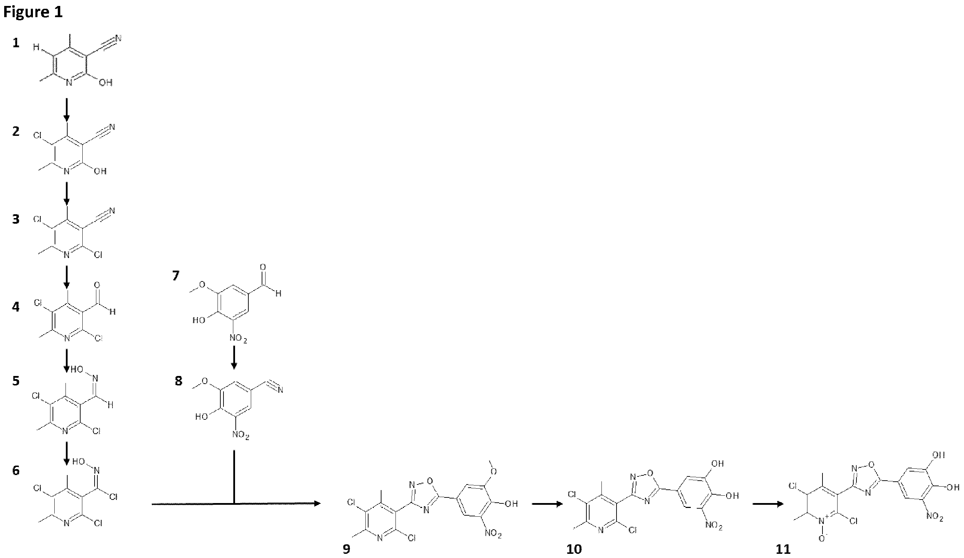

Figure 1 exhibits one possible path of reaction for obtaining Opicapone. The central reaction is the formation of the oxadiazol-ring between compound 6 and compound 8. The intermediate compounds 1-5 display one possible route of synthesis for the formation of compound 6, whereas compound 8 can be directly synthesized starting form compound 7. After formation of the oxadiazol-moiety in compound 9 the compound is firstly de-methylated (compound 10) and in a second step oxidized to yield the final product Opicapone (compound 11).

### EXPERIMENTAL EXAMPLES

### Reaction step 1:

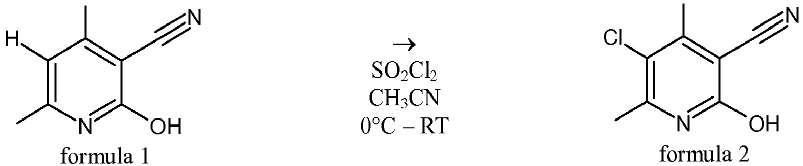

4,6-dimethyl-2-hydroxynicotinonitrile (1 g, 6.61 mmol) was suspended in CH₃CN (6 ml) under nitrogen atmosphere and the suspension was cooled with an ice bath. Sulfuryl chloride was added dropwise. Another 4 ml CH₃CN was added and the suspension was stirred at 0 °C for 30 minutes. Afterwards it was allowed to warm to room temperature and was stirred for 5 hours. The suspension was cooled and the precipitate was isolated and washed with additional CH₃CN. 5-chloro-2-hydroxy-4,6-dimethylnicotinonitrile (0.887 g, 4.86 mmol) was isolated as a white solid, yield = 74 %.
¹H-NMR (DMSO-d6): 12.801 (b, 1H); 2.41 (s, 3 H, CH₃); 2.35 (s, 3 H, CH₃). ¹³C-NMR (DMSO-d6): 159.27 (arom. C); 157.55 (arom. C); 149.41 (arom. C); 115.53 (C, CN); 111.2 (arom. C); 100.92 (arom. C); 20.05 (C, CH3); 18.22 (C, CH3).

### Reaction step 2:

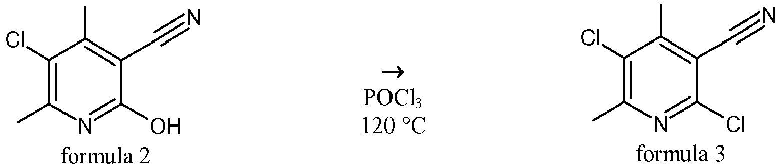

A Büchi glasreactor was loaded with 5-chloro-2-hydroxy-4,6-dimethylnicotinonitrile (4.000 g, 21.91 mmol) and POCl₃ (3.358 g, 21.91 mmol, 2.04 ml). The mixture was heated to 120 °C and stirred for 6 hours. Ice and ice water was added to the mixture and the precipitate was isolated and washed with ice water. 2,5-dichloro-4,6-dimethylnicotinonitrile (3.373 g, 16.78 mmol) was isolated as an off-white solid, yield = 77 %.
¹H-NMR (DMSO-d6): 2.61 (s, 3H, CH₃); 2.56 (s, 3H, CH₃)
¹³C-NMR (DMSO-d6): 160.19 (arom. C); 152.35 (arom. C); 148.23 (arom. C); 130.38 (arom. C); 114.10 (C, CN); 109.39 (arom. C); 23.30 (C, CH3); 19.69 (C, CH3).

### Reaction step 3:

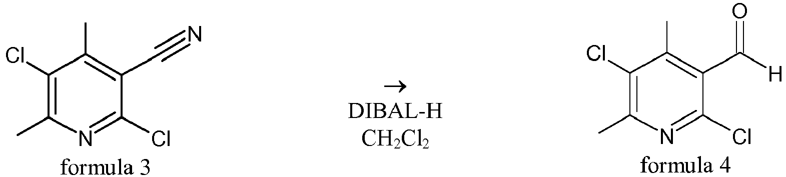

A 50 ml Schlenk flask was loaded with 2,5-dichloro-4,6-dimethylnicotinonitrile (1.000 g, 4.97 mmol) and CH₂Cl₂ (dry, 8 ml) and the mixture was cooled with an ice/NaCl bath. DIBAL-H solution (1.2 M in toluene, 4.35 ml, 4.97 mmol) was added dropwise to the solution. The mixture was allowed to warm to room temperature and after 5 hours additional DIBAL-H solution (1 ml) was added. After 2 hours, the reaction was quenched with water and the pH was adjusted to 1 with HCl (conc.). The mixture was heated to reflux for 2 hours. The mixture was extracted with EtOAc and the organic phase was washed with NaHCO₃ (sat. solution) and brine, dried over MgSO₄ and the solvent was evaporated. 2,5-dichloro-4,6-dimethylnicotinaldehyde (0.8224 g, 4.03 mmol) was isolated as an off-white solid, yield = 81 %.
¹H-NMR (CDCl₃): 10.49 (s, 1H, CHO); 2.67 (d, 6 H, 2 x CH₃).
¹³C-NMR (CDCl₃): 190.13 (C, CHO); 160.58 (arom. C); 151.25 (arom. C); 149.91 (arom. C); 132.90 (arom. C); 129.36 (arom. C); 24.11 (C, CH3); 17.14 (C, CH3).

### Reaction step 4:

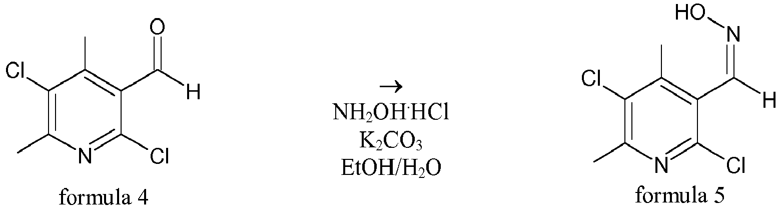

2,5-dichloro-4,6-dimethylnicotinaldehyde (700 mg, 3.43 mmol) was suspended in a mixture of EtOH (10 ml) and water (1 ml). K₂CO₃ (711.15 mg, 5.15 mmol, 1.5 eq.) and NH₂OH. HCl (286.06 mg, 4.11 mmol, 1.2 eq.) was added and the suspension was stirred at room temperature for 90 minutes. Afterwards, the solvent was evaporated and the residue was extracted with EtOAc, yielding 2,5-dichloro-4,6-dimethylnicotinaldehyde oxime (670 mg, 3.06 mmol) as an off-white solid, yield = 89 %. ¹H-NMR (CDCl₃): 8.39 (s, 1H, CHN); 2.63 (s, 3H, CH3); 2.56 (s, 3H, CH3).
¹³C-NMR (CDCl₃): 156.72 (arom. C); 147.86 (C, NHC); 147.65 (arom. C); 147.38 (arom. C); 132.02 (arom. C); 124.64 (arom. C); 23.46 (C, CH3); 19.03 (C, CH3).

### Reaction step 5:

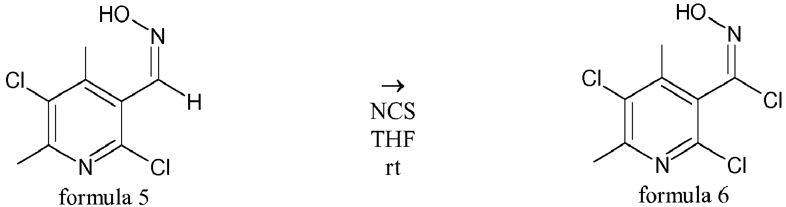

2,5-dichloro-4,6-dimethylnicotinaldehyde oxime (1.69 g, 7.71 mmol) was dissolved in THF (dry, 18 ml) under nitrogen atmosphere. NCS (1.13 g, 8.48 mmol, 1.1 eq) was added portion-wise and the mixture was stirred at room temperature for 2.5 hours. The mixture was quenched with water and was extracted with EtOAc. The organic phase was washed with brine, dried over MgSO₄ and concentrated, yielding 2,5-dichloro-N-hydroxy-4,6- dimethylnicotinimidoyl chloride (1.71 g, 6.73 mmol) as a pale-yellow solid, yield = 87 %.
¹H-NMR (DMSO-d6): 12.77 (s, 1H, OH); 2.58 (s, 3H, CH3); 2.35 (s, 3H; CH3). ¹³C-NMR (DMSO-d6): 179.37; 157.46; 147.92; 146.10; 130.53; 128.86; 127.89; 22.85 (C, CH3); 17.84 (C, CH3).

### Reaction step 6:

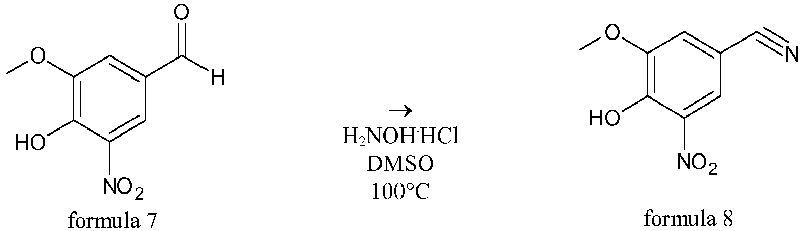

5-Nitrovaniline (10.00 g, 50.73 mmol) and hydroxylamine hydrochloride (6.52 g, 93.86 mmol, 1.85 eq.) were dissolved in DMSO (100 ml). The mixture was stirred at 100 °C for 2 hours. Afterwards, the mixture was added dropwise into excess water and the formed precipitate was isolated. 4-hydroxy-3-mehtoxy-5-nitrobenzonitrile (8.57 g, 44.14 mmol) was collected as a yellow powder, yield = 87 %.
¹H-NMR (DMSO-d6): 8.00 (d, J = 2 Hz, 1H, arom. H); 7.67 (d, J = 1.5 Hz, 1H, arom. H); 3.92 (s, 3H, CH3).
¹³C-NMR (DMSO-d6): 150.08 (arom. C); 146.43 (arom. C); 137.60 (arom. C); 121.69 (arom. C); 117.90 (C, CN); 117.80 (arom. C); 100.61 (arom. C); 57.27 (C, CH3).

### Reaction step 7:

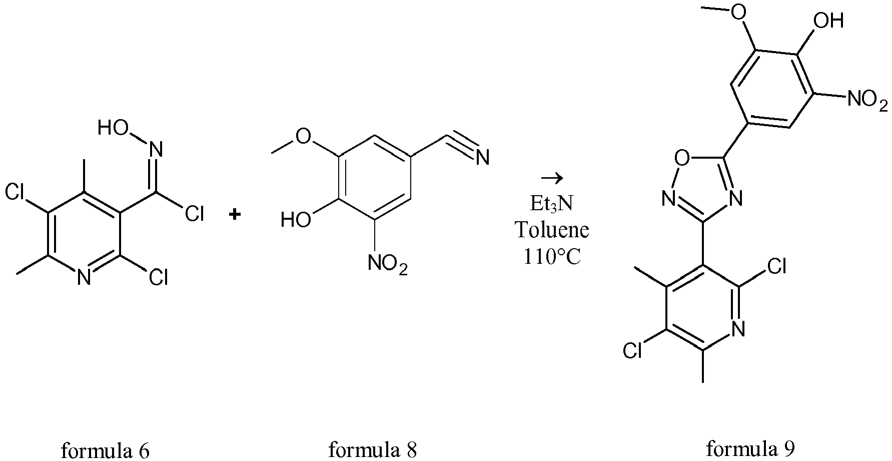

2,5-dichloro-N-hydroxy-4,6-dimethylnicotinimidoyl chloride (1.00 g, 3.9 mmol, 1.5 eq.) and 4- hydroxy-3-mehtoxy-5-nitrobenzonitrile (510.54 mg, 2.63 mmol) were dissolved in toluene (dry, 9 ml) and the mixture was heated to 110 °C. Diluted Et₃N (399.15 mg, 1.18 mmol, 1.5 eq, in 10 ml toluene) was added over 2 hours to the mixture and the solution was stirred for 2 hours. The solvent was evaporated and little EtOH was added to the residue, leading to a precipitate that was collected. 4-(3-(2,5-dichloro-4,6-dimethylpyridin-3-yl)-1,2,4-oxadiazol-5-yl)-2-methoxy-6-nitrophenol (0.54 g, 1.31 mmol) was isolated as a yellow powder, yield = 50%. ¹H-NMR (DMSO-d6): 8.25 (d, J = 2 Hz, 1H, arom. H); 7.84 (d, J = 2 Hz, 1H, arom. H); 4.02 (s, 3H, OCH3); 2.64 (s, 3H, CH3); 2.29 (s, 3H, CH3).
¹³C-NMR (DMSO-d6): 174.94 (C, NCO); 165.23 (C, NCN); 158.18 (arom. C); 150.41 (arom. C); 148.79 (arom. C); 147.11 (arom. C); 146.26 (arom. C); 137.52 (arom. C); 130.74 (arom. C); 121.52 (arom. C); 117.17 (arom. C); 113.37 (arom. C); 112.48 (arom. C); 57.04 (C, OCH3); 22.95 (C, CH3); 18.38 (C, CH3).

### Reaction step 8:

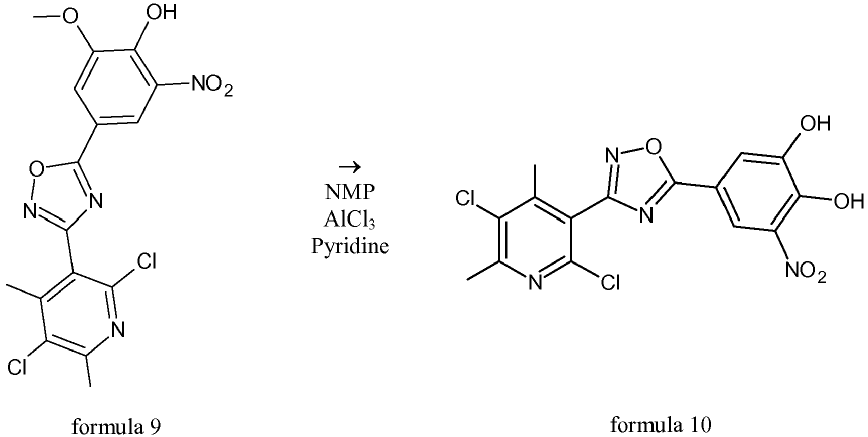

A solution of 4-(3-(2,5-dichloro-4,6-dimethylpyridin-3-yl)-1,2,4-oxadiazol-5-yl)-2-methoxy-6- nitrophenol (200 mg, 0.49 mmol) in NMP (2 ml) was cooled in an ice bath. AlCl₃ (85.61 mg, 0.64 mmol, 1.32 eq.) and Pyridine (166.20 mg, 2.10 mmol, 4.32 eq.) were added. Afterwards the mixture was heated to 60 °C and stirred for 2 hours. The reaction was cooled with an ice bath and quenched with H₂O/HCl (2.7 ml/ 1.2 ml (conc.)). The precipitate was isolated and washed with water. 5-(3-(2,5-dichloro-4,6-dimethylpyridin-3-yl)-1,2,4-oxadiazol-5-yl)-3-nitrobenzene-1,2-diol was isolated as a yellow solid (179.8 mg, 0.45 mmol), yield = 93 %.
¹H-NMR (DMSO-d6): 8.11 (1H, arom. H); 7.76 (1H, arom. H); 2.69 (s, 3H, CH3); 2.29 (s, 3H, CH3).
¹³C-NMR (DMSO-d6): 174.85 (C, NCO); 165.17 (C, NCN); 158.15 (arom. C); 148.83 (arom. C); 148.65 (arom. C); 146.27 (arom. C); 146.07 (arom. C); 137.83 (arom. C); 130.77 (arom. C); 121.54 (arom. C); 116.71 (arom. C); 115.49 (arom. C); 112.87 (arom. C); 22.95 (C, CH3); 18.37 (C, CH3).

### Alternative Reaction Step 8:

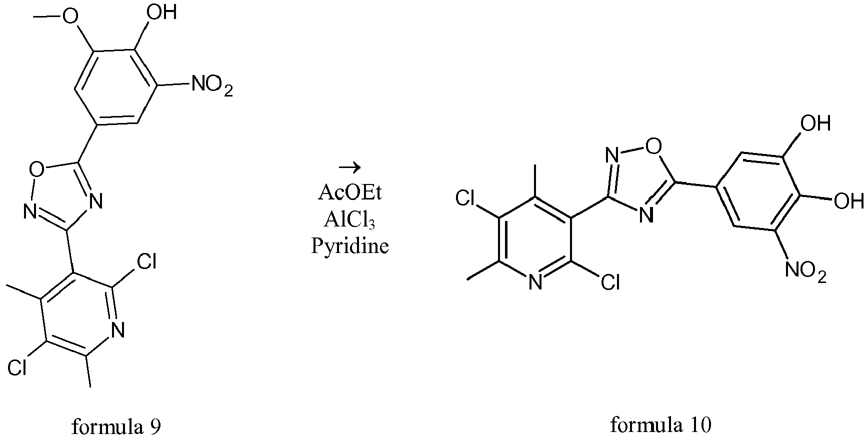

In a 25 mL single neck round bottomed flask, to a stirred yellow solution 4-(3-(2,5-dichloro-4,6-dimethylpyridin-3-yl)-1,2,4-oxadiazol-5-yl)-2-methoxy-6-nitrophenol (30 mg, 0.073 mmol) in ethyl acetate (2 mL) at room temperature was added aluminum chloride (12 mg, 0.09 mmol) in one portion. To the resulting orange/red suspension pyridine (24 mg 0.29 mmol, 0.025 mL) was added dropwise, causing the internal temperature to rise to 45 °C. The orange solution was then heated at 60 °C for 2 hours, then at 70°C for another hour. After the allotted time, the color of the solution switched to light orange. Whereupon ice was carefully added to the reaction mixture followed by concentrated hydrochloric acid (1 mL). After being stirred at 50 °C for 1 h, the volatiles were removed and the yellow solid was triturated in AcOEt (10 mL) and then filtered to afford the product. Yield= 55%.
¹H-NMR (DMSO-d6): 8.11 (1H, arom. H); 7.76 (1H, arom. H); 2.69 (s, 3H, CH3); 2.29 (s, 3H, CH3).
¹³C-NMR (DMSO-d6): 174.85 (C, NCO); 165.17 (C, NCN); 158.15 (arom. C); 148.83 (arom. C); 148.65 (arom. C); 146.27 (arom. C); 146.07 (arom. C); 137.83 (arom. C); 130.77 (arom. C); 121.54 (arom. C); 116.71 (arom. C); 115.49 (arom. C); 112.87 (arom. C); 22.95 (C, CH3); 18.37 (C, CH3).

### Alternative Reaction Step 8:

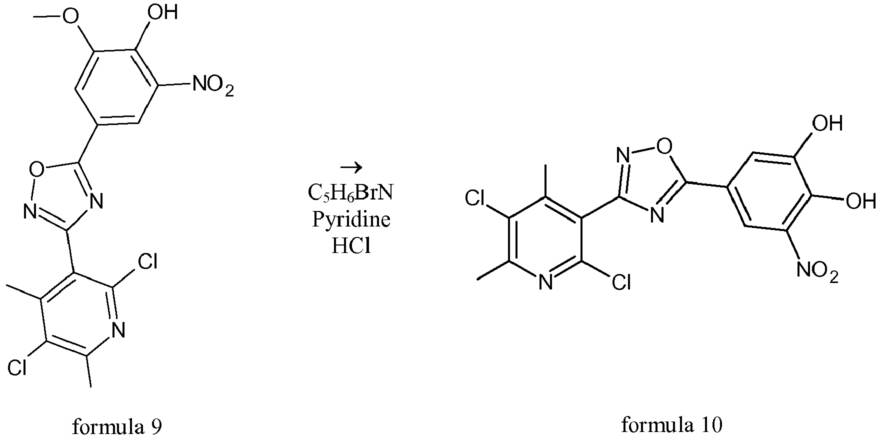

In a 25 mL Young Schlenk, was weighed the 4-(3-(2,5-dichloro-4,6-dimethylpyridin-3-yl)-1,2,4-oxadiazol-5-yl)-2-methoxy-6-nitrophenol (20 mg, 0.049 mmol) and pyridine hydrobromide (C₅H₆BrN) (9 mg, 0.22 mmol) was added in one portion. The Schlenk was evacuated and backfilled with nitrogen. Then, pyridine (2 mL) was added. The reaction mixture was stirred at 130°C and followed by UHPLC analysis. After the allotted time, the reaction mixture was allowed to cool to room temperature, it was acidified with 1 N HCl (ca. 10 mL) and extracted with AcOEt (15 mL×2) and the combined organic layers were washed with water and brine, dried over Mg₂SO₄ and finally concentrated in vacuo to afford the wanted product. Yield=87.5%. A benefit of this alternative reaction step is the avoidance of aluminum salts and a simplified isolation of the reaction product.
¹H-NMR (DMSO-d6): 8.11 (1H, arom. H); 7.76 (1H, arom. H); 2.69 (s, 3H, CH3); 2.29 (s, 3H, CH3).
¹³C-NMR (DMSO-d6): 174.85 (C, NCO); 165.17 (C, NCN); 158.15 (arom. C); 148.83 (arom. C); 148.65 (arom. C); 146.27 (arom. C); 146.07 (arom. C); 137.83 (arom. C); 130.77 (arom. C); 121.54 (arom. C); 116.71 (arom. C); 115.49 (arom. C); 112.87 (arom. C); 22.95 (C, CH3); 18.37 (C, CH3).

### Reaction step 9:

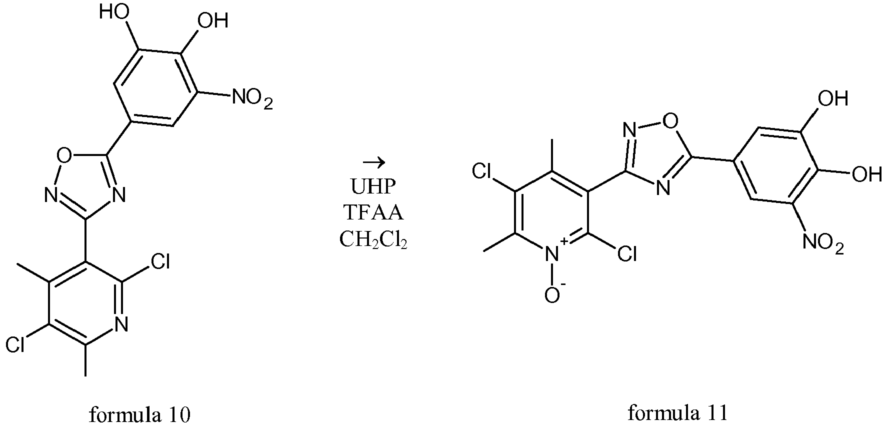

A suspension of 5-(3-(2,5-dichloro-4,6-dimethylpyridin-3-yl)-1,2,4-oxadiazol-5-yl)-3-nitrobenzene-1,2-diol (70 mg, 0.17 mmol, 1 eq.) in CH₂Cl₂ (1.5 ml) was cooled with an ice bath. Urea hydrogen peroxide (53.05 mg, 0.56 mmol, 3.2 eq.) and trifluoroacetic anhydride (111.05 mg, 0.53 mmol, 3 eq.) were added and the mixture was allowed to reach room temperature overnight. The reaction mixture was filtered and washed with CH₂Cl₂. The filtrate was washed with water. The combined organic phases were dried with MgSO₄ and the solvent was evaporated.
¹H-NMR (DMSO-d6): 8.11 (arom. H); 7.73 (arom. H); 2.66 (CH3); 2.24 (CH3).
¹³C-NMR (DMSO-d6): 175.15 (C, NCO); 164.50 (C, NCN); 150.31 (arom. C); 148.66 (arom. C); 146.27 (arom. C); 139.36 (arom. C); 137.79 (arom. C); 134.06 (arom. C); 131.03 (arom. C); 122.68 (arom. C); 116.64 (arom. C); 115.61 (arom. C); 112.80 (arom. C); 17.87 (C, CH3); 16.47 (C, CH3).

### Alternative Reaction Step 9:

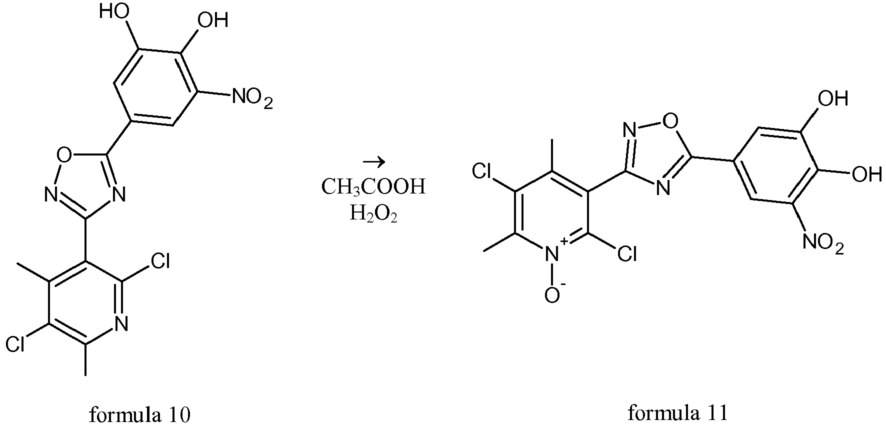

In a 25 mL round bottomed flask, to a solution of 5-(3-(2,5-dichloro-4,6-dimethylpyridin-3-yl)-1,2,4-oxadiazol-5-yl)-3-nitrobenzene-1,2-diol (16.2 mg, 0.041 mmol) in glacial acetic acid (14.3 g) was added hydrogen peroxide 35% (72 mg). The solution was stirred overnight at room temperature. Then all the volatiles were removed by means of rotary evaporation and the white-off solid was dried under high vacuum to afford 17 mg of the wanted product with a purity of 95%.
¹H-NMR (DMSO-d6): 8.11 (arom. H); 7.73 (arom. H); 2.66 (CH3); 2.24 (CH3).
¹³C-NMR (DMSO-d6): 175.15 (C, NCO); 164.50 (C, NCN); 150.31 (arom. C); 148.66 (arom. C); 146.27 (arom. C); 139.36 (arom. C); 137.79 (arom. C); 134.06 (arom. C); 131.03 (arom. C); 122.68 (arom. C); 116.64 (arom. C); 115.61 (arom. C); 112.80 (arom. C); 17.87 (C, CH3); 16.47 (C, CH3).

## Claims

1. Process for the preparation of Opicapone ((4Z)-4-[3-(2,5-dichloro-4,6-dimethyl-1-oxidopyridin-1-ium-3-yl)-2H-1,2,4-oxadiazol-5-ylidene]-2-hydroxy-6-nitrocyclohexa-2,5-dien-1-one) according to the following formula 11 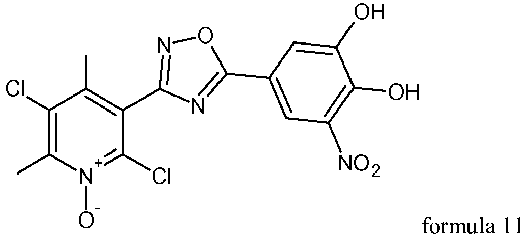 or a pharmaceutically acceptable salt thereof, **characterized in that** the process at least comprises the reaction of a compound according to the following formula 6 (2,5-dichloro-N-hydroxy-4,6-dimethylnicotinimidoyl chloride) 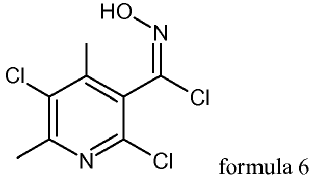 and a compound according to the following formula 8 (4-hydroxy-3-methoxy-5-nitrobenzonitrile) 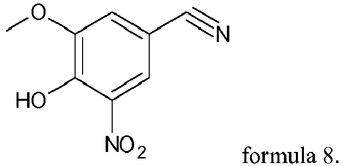 in the presence of a solvent and a base.

2. Process according to claim 1, wherein the reaction of the compound according to formula 6 and formula 8 is performed at a temperature of ≥ 80°C and ≤ 140°C.

3. Process according to any of claims 1 to 2, wherein the reaction of the compound according to formula 6 and formula 8 is performed in one or more aromatic solvents comprising a boiling point of ≥ 80°C.

4. Process according to any of claims 1 to 3, wherein the base is a tertiary amine.

5. Process according to claim 4, wherein the tertiary amine is NEt₃.

6. Process according to any of claims 1 to 5, wherein the reaction product of the reaction between a compound according to formula 6 and a compound according to formula 8 is a compound according to formula 9 (4-(3-(2,5-dichloro-4,6-dimethylpyridin-3-yl)-1,2,4-oxadiazol-5-yl)-2-methoxy-6-nitrophenol) 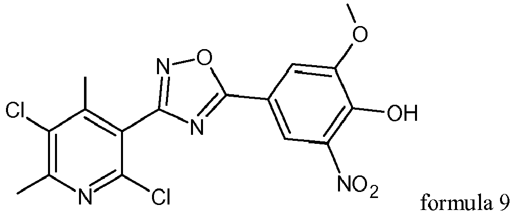 and the compound according to formula 9 is de-methylated to yield a compound according to formula 10 (5-(3-(2,5-dichloro-4,6-dimethylpyridin-3-yl)-1,2,4-oxadiazol-5-yl)-3-nitrobenzene-1,2-diol) 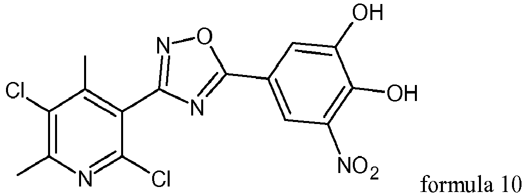 followed by N-oxidation to yield Opicapone according to formula 11.

7. Process according to claim 6, wherein the oxidation is performed in dichloromethane, chloroform, ethyl acetate, or mixtures, or in the presence of pyridine hydrobromide thereof.

8. Process according to claim 6, wherein the oxidation is performed in the presence of hydrogen peroxide and trifluoroacetic anhydride at room temperature.

9. Process according to any of claims 1 to 8, wherein the compound according to formula 6 is prepared via the steps of
a) reacting 4,6-dimethyl-2-hydroxynicotinonitrile and a chlorinating agent to yield 2,5-dichloro-4,6-dimethylnicotinonitrile 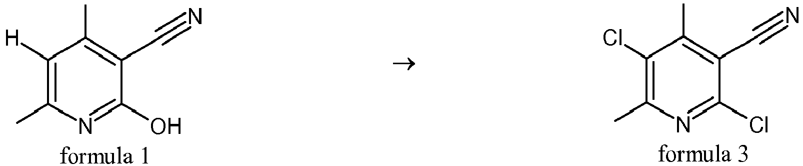
b) reacting the 2,5-dichloro-4,6-dimethylnicotinonitrile obtained in step a) in the presence of a reducing agent to yield 2,5-dichloro-4,6-dimethylnicotin-aldehyde 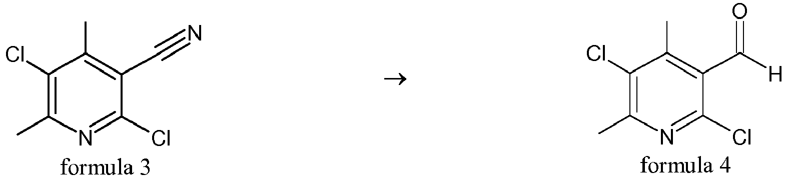
c) reacting the 2,5-dichloro-4,6-dimethylnicotinaldehyde obtained in step b) with hydroxylamine to yield 2,5-dichloro-4,6-dimethylnicotinaldehyde-oxime 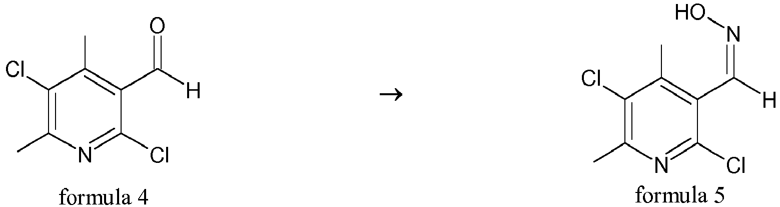
d) reacting the 2,5-dichloro-4,6-dimethylnicotinaldehyde-oxime obtained in step c) and a chlorinating agent to yield compound 5 2,5-dichloro-N-hydroxy-4,6-dimethylnicotinimidoyl-chloride

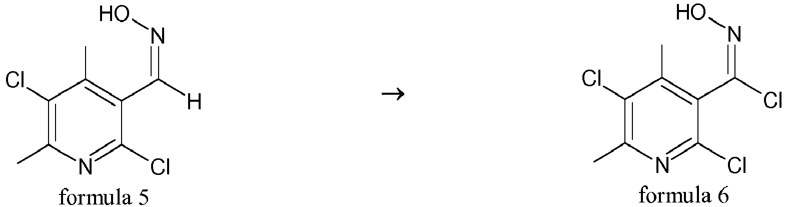

10. Process according to claim 9, wherein the chlorinating step a) is performed stepwise, wherein in a first reaction step
a1) 4,6-dimethyl-2-hydroxynicotinonitrile is mono-chlorinated in 5 position in the presence of sulfuryl chloride to yield 5-chloro-2-hydroxy-4,6-dimethylnicotinonitrile 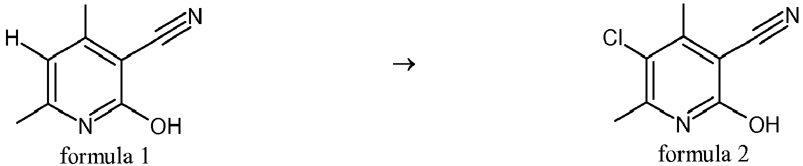 and in a second step
a2) 5-chloro-2-hydroxy-4,6-dimethylnicotinonitrile is chlorinated in the presence of phosphorus oxychloride to 2,5-chloro-4,6-dimethylnicotinonitrile

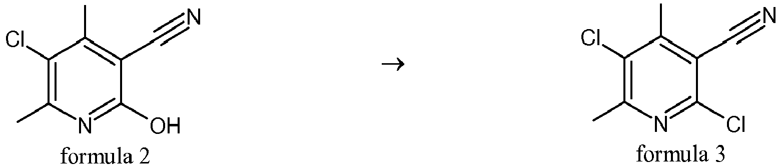

11. Process according to any of claims 1 to 10, wherein the compound according to formula 8 is prepared via reaction of 5-Nitrovaniline and hydroxylamine hydrochloride to yield the compound according to formula 8

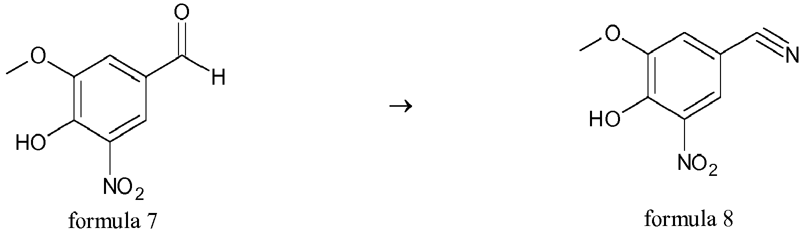

12. Process according to any of claims 1 to 11, wherein all reaction steps in the preparation of the compound according to formula 6 and all reaction steps in the preparation of the compound according to formula 8 are performed below 50°C.

13. Compound (2,5-dichloro-N-hydroxy-4,6-dimethylpyridine-3-carboximidoyl chloride) according to the following formula 6 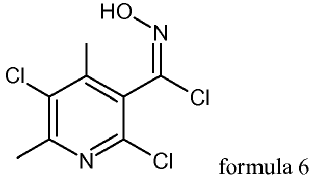 or a salt thereof.

14. Intermediate in the production of Opicapone at least comprising a compound according to the following formula 6 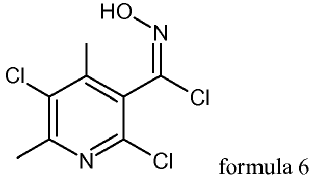 or a pharmaceutically acceptable salt thereof.

15. Intermediate in the production of Opicapone at least comprising a compound according to the following formula 8 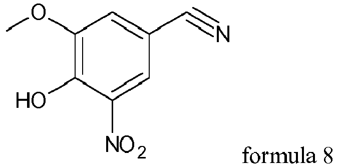 or a pharmaceutically acceptable salt thereof.

16. Pharmaceutical composition comprising Opicapone synthesized according to any of claims 1-15 in combination with a pharmaceutically acceptable carrier.

17. Use of a pharmaceutical composition according to claim 16 for the treatment of central or peripheral nervous system disorders.
